# EUROPÄISCHE PATENTANMELDUNG

(11) **EP 2 110 105 A1**
(43) Veröffentlichungstag der Anmeldung: **21.10.2009**
(21) Anmeldenummer: 09005442.0
(22) Anmeldetag: 16.04.2009
(51) Int. Cl.: A61F 5/37, A61B 19/00

(54) **Fixiervorrichtung für den kopfnahen Oberkörperbereich einer Person**

(30) Priorität: 18.04.2008 DE 202008005405 U
(71) Anmelder: Back, Robert, 76829 Landau (DE)
(72) Erfinder: Back, Robert, 76829 Landau (DE)
(74) Vertreter: Zeitler - Volpert - Kandlbinder

(57) **Zusammenfassung**

Bei einer Fixiervorrichtung für den kopfnahen Oberkörperbereich einer Person, insbesondere für deren Kopf und/oder Hals und/oder Nacken und/oder Schultern, ist die Anordnung derart getroffen, dass ein etwa U-förmiges Auflageteil (1) für den kopfnahen Oberkörperbereich, dessen beide U-Schenkel (2) zur Bildung eines Schulterauflagebereichs keilförmig ansteigend ausgestaltet sind und dessen die U-Schenkel (2) verbindender Quersteg (3) einen keilförmig ansteigenden Hals/Nackenbereich (3a) sowie einen daran anschließenden Kopfauflagebereich (3b) bildet, zwei auf der Höhe des Kopfauflagebereiches (3b) im Abstand voneinander vorgesehene Seitenwangen (4) zur seitlichen Fixierung des Kopfes und ein die Seitenwangen (4) lösbar bzw. verstellbar überspannendes Band (6) zur vertikalen Fixierung des Kopfes.

## Beschreibung

Die Erfindung betrifft eine Fixiervorrichtung für den kopfnahen Oberkörperbereich einer Person, insbesondere für deren Kopf und/oder Hals und/oder Nacken und/oder Schultern gemäß dem Oberbegriff des Anspruchs 1.

Bekannte Fixiervorrichtungen der gattungsgemäßen Art (US 2006/0249163 A1) kommen in vielen Fällen zur Anwendung und dienen vor allen Dingen dazu, den kopfnahen Oberkörperbereich einer verletzten Person, also insbesondere deren Kopf und/oder Hals und/oder Nacken und/oder Schultern, derart zu fixieren, dass diese Person transportiert und auch medizinisch behandelt werden kann, ohne dass weitere Verletzungen bzw. Schädigungen auftreten. Ein besonderer Anwendungsbereich ist hierbei der Motorradrennsport, wobei es bei auftretenden Verletzungen besonders darauf ankommt, die verletzte Person in außerordentlich kurzer Zeit - bei fixiertem körpernahem Oberkörperbereich - dem erforderlichen Transport und/oder der erwünschten Behandlung zuzuführen.

Die oben erwähnte bekannte Fixiervorrichtung weist zwar zur Fixierung des Kopfes ein Kopfaufnahmeteil sowie hieran vorderseitig anschließend ein Nacken- bzw. Schulterauflageteil auf. Nachteilig ist hierbei jedoch, dass die betreffenden Auflageteile starr ausgebildet und daher nicht an unterschiedlich große Patienten anpassbar sind, da die jeweiligen Auflageteile seitlich durch starre Bügel begrenzt sind.

Der Erfindung liegt daher die Aufgabe zugrunde, zur Beseitigung der geschilderten Nachteile die Fixiervorrichtung der gattungsgemäßen Art derart auszugestalten, dass sie bei einfacher Herstellung die ihr zugedachte Funktion wirkungsvoll sowie präzise erfüllt und außerdem sehr flexibel an unterschiedliche Abmessungen der zu fixierenden Person anpassbar ist.

Diese Aufgabe wird durch die Erfindung mit den Merkmalen des Anspruchs 1 gelöst. Vorteilhafte Ausgestaltungen hiervon sind in den weiteren Ansprüchen beschrieben.

Die erfindungsgemäße Fixiervorrichtung zeichnet sich aus durch ein etwa U-förmiges Auflageteil für den kopfnahen Oberkörperbereich, dessen beide U-Schenkel zur Bildung eines Schulterauflagebereiches keilförmig ansteigend ausgestaltet sind und dessen die U-Schenkel verbindender Quersteg einen keilförmig ansteigenden Hals-/Nackenbereich sowie einen daran anschließenden Kopfauflagebereich bildet; weiterhin sind zwei auf der Höhe des Kopfauflagebereiches im Abstand voneinander angeordnete Seitenwangen zur seitlichen Fixierung des Kopfes vorgesehen; schließlich beinhaltet die Erfindung auch ein die Seitenwangen lösbar bzw. verstellbar überspannendes Band zur vertikalen Fixierung des Kopfes.

Die erfindungsgemäße Fixiervorrichtung weist den Vorteil auf, dass sie bei einfacher Herstellung gleichwohl außerordentlich leicht und einfach an unterschiedliche Abmessungen der zu fixierenden Person anpassbar ist. Gleichzeitig ist eine präzise Lagerung und Fixierung der verletzten Person bzw. deren Kopf, Hals, Nacken und Schultern gewährleistet.

In Weiterbildung der Erfindung ist vorgesehen, dass die den Schulterauflagebereich bildenden U-Schenkel etwa bis zum Beginn des Quersteges keilförmig ansteigend verlaufen.

Es liegt im Rahmen der Erfindung, dass der Hals-/Nackenauflagebereich eine größere Steigung als der Schulterauflagebereich aufweist.

Es ist von Vorteil, wenn der Kopfauflagebereich oberseitig weitgehend eben verläuft.

Zweckmäßigerweise sind die Seitenwangen beidseits des Kopfauflagebereiches lösbar am Auflageteil festlegbar. Zu diesem Zweck können die Seitenwangen vorteilhafterweise mittels Klettverschluss beidseits des Kopfauflagebereichs festlegbar sein.

Vorzugsweise sind die Seitenwangen quaderförmig ausgebildet, können jedoch bei Bedarf auch jede andere geeignete Form aufweisen.

Besondere Vorteile ergeben sich, wenn das Band zur vertikalen Fixierung des Kopfes seitlich am Auflageteil, vorzugsweise mittels Klettverschluss, festlegbar ist. Hierdurch lässt sich das Band mit jeder gewünschten Länge bzw. Einstellung festlegen.

Es liegt im Rahmen der Erfindung, dass die Fixiervorrichtung aus einem Kunststoffschaum besteht, der mit einem Überzug, insbesondere Kunstleder, und einem Feuchtigkeitsschutzbezug versehen ist. Als Kunststoffschaum kann beisp. ein unter dem Handelsnamen "Neopolen" erhältlicher Polypropylen - Schaumstoff zur Anwendung gelangen, der aus expandierten, vorwiegend geschlossenzelligen Schaumstoffpartikeln gebildet wird.

Die Erfindung hat einen außerordentlich weiten Einsatzbereich, beisp. im Motorradrennsport, Straßenrennsport, Moto Cross usw. und natürlich auch abseits von Rennstrecken, also im normalen Straßenverkehr.

Die erfindungsgemäße Fixiervorrichtung lässt sich leicht und einfach auf unterschiedliche Größen der betreffenden Körperteile von Kindern, Jugendlichen und Erwachsenen einstellen und kann insbesondere auch verwendet werden in Verbindung mit dem an Motorrad - Lederkombinationen rückenseitig integrierten aerodynamischen Aufsatz, auch "HUMP" genannt.

Die Erfindung wird im Folgenden in Form eines Ausführungsbeispiels anhand der Zeichnung näher erläutert. Hierbei zeigen:

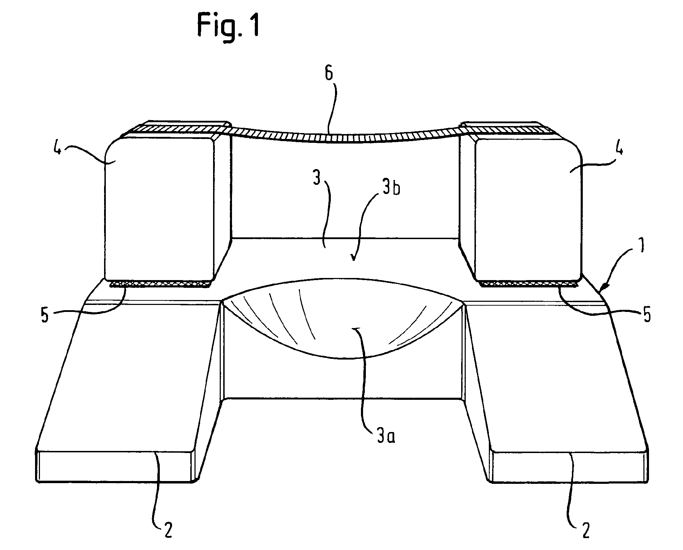

- Fig. 1: die Fixiervorrichtung gemäß der Erfindung perspektivisch in Vorderansicht und
- Fig. 2: perspektivisch in Seitenansicht.

Die aus der Zeichnung ersichtliche Fixiervorrichtung dient zum Immobilisieren des kopfnahen Oberkörperbereichs einer Person, insbesondere für deren Kopf und/oder Hals und/oder Nacken und/oder Schultern, d.h. also um bei einer verletzten Person oder einem Patienten den Schädel sowie die Halswirbelsäule (HWS) einschließlich des oberen Thoraxbereiches mit Schultern in der erwünschten achsgerechten Position zu immobilisieren.

Wie dargestellt, weist die Fixiervorrichtung ein Auflageteil 1 auf, das etwa U-förmig mit zwei U-Schenkeln 2 sowie einem diese verbindenden Quersteg 3 ausgebildet ist. Hierbei bilden die U-Schenkel 2 einen Schulterauflagebereich, während der Quersteg 3 einen Hals-/Nackenauflagebereich 3a einschließlich sich eines daran nach hinten anschließenden Kopfauflagebereichs 3b bildet.

Wie ersichtlich, sind die den Schulterauflagebereich bildenden beiden U-Schenkel 2 keilförmig ansteigend ausgestaltet, wobei diese Steigung sich etwa bis zum Beginn des Quersteges 3 erstreckt.

Der zwischen den beiden U-Schenkein 2 am Quersteg 3 gebildete Hals-/Nackenauflagebereich 3a verläuft ebenfalls keilförmig ansteigend, jedoch - wie dargestellt - mit einer größeren Steigung als die den Schulterauflagebereich bildenden U-Schenkel 2. Dies sorgt für eine passgerechte Lagerung sowohl des oberen Thoraxbereiches einschließlich der Schultern der Person mittels der U-Schenkel 2 als auch der Halswirbelsäule mittels des Hals-/Nackenauflagebereichs 3a.

Der anschließend an den Hals-/Nackenauflagebereich 3a im Quersteg 3 gebildete Kopfauflagebereich 3b verläuft oberseitig weitgehend eben, so dass unabhängig von der jeweiligen Kopfform der betreffenden Person stets eine gleich bleibend zutreffende Lagerung des Kopfes gewährleistet ist.

Zur seitlichen Fixierung des Kopfes sind auf der Höhe des Kopfauflagebereiches 3b zwei Seitenwangen 4 vorgesehen. Diese sind etwa quaderförmig ausgebildet und im Abstand voneinander angeordnet. Hierbei können diese Seitenwangen 4 beidseits des Kopfauflagebereiches 3b lösbar am Auflageteil 1 bzw. an dessen Quersteg 3 im jeweils gewünschten Abstand festgelegt werden, und zwar im dargestellten Ausführungsbeispiel mittels eines angedeutet gezeichneten Klettverschlusses 5, der mit einem jeweils an der Unterseite der Seitenwangen 4 vorgesehenen entsprechenden Klettverschlussband zusammenwirkt. Hierdurch kann der Kopf der jeweiligen Person in einfacher und schneller Weise mittels der Seitenwangen 4 seitlich fixiert werden.

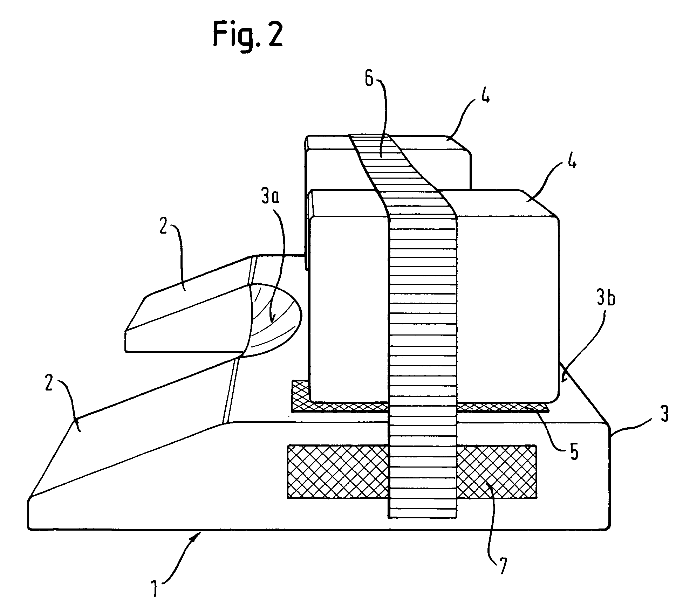

Zur vertikalen Fixierung des Kopfes ist schließlich ein Band 6 vorgesehen, das etwa auf der Höhe der Stirn der betreffenden Person quer über die Seitenwangen 4 gespannt und gleichfalls mittels Klettverschluss 7 (siehe Fig. 2), der sich beidseits seitlich am Quersteg 3 des Auflageteils 1 befindet, festgelegt werden kann.

Das Auflageteil 1 sowie die Seitenwangen 4 der Fixiervorrichtung sind vorzugsweise aus einem Schaumstoff, beispielsweise erhältlich unter dem Handelsnamen Polyform, gebildet, der mit einem Überzug, insbesondere Kunstleder, versehen ist.

Hinsichtlich vorstehend nicht im einzelnen erläuterter Merkmale der Erfindung wird ausdrücklich auf die Zeichnung sowie die Ansprüche verwiesen.

## Patentansprüche

1. Fixiervorrichtung für den kopfnahen Oberkörperbereich einer Person, insbesondere für deren Kopf und/oder Hals und/oder Nacken und/oder Schultern,
**gekennzeichnet durch**
- ein etwa U-förmiges Auflageteil (1) für den kopfnahen Oberkörperbereich, dessen beide U-Schenkel (2) zur Bildung eines Schulterauflagebereichs keilförmig ansteigend ausgestaltet sind und dessen die U-Schenkel (2) verbindender Quersteg (3) einen keilförmig ansteigenden Hals-/Nackenbereich (3a) sowie einen daran anschließenden Kopfauflagebereich (3b) bildet,
- zwei auf der Höhe des Kopfauflagebereiches (3b) im Abstand voneinander vorgesehene Seitenwangen (4) zur seitlichen Fixierung des Kopfes und
- ein die Seitenwangen (4) lösbar bzw. verstellbar überspannendes Band (6) zur vertikalen Fixierung des Kopfes.

2. Vorrichtung nach Anspruch 1, **dadurch gekennzeichnet, dass** die den Schulterauflagebereich bildenden U-Schenkel (2) bis zum Beginn des Quersteges (3) keilförmig ansteigend verlaufen.

3. Vorrichtung nach Anspruch 1 oder 2, **dadurch gekennzeichnet, dass** der Hals-/Nackenauflagebereich (3a) des Quersteges (3) eine größere Steigung als die den Schulterauflagebereich bildenden U-Schenkel (2) aufweist.

4. Vorrichtung nach einem der vorhergehenden Ansprüche, **dadurch gekennzeichnet, dass** der Kopfauflagebereich (3b) oberseitig weitgehend eben verläuft.

5. Vorrichtung nach einem der vorhergehenden Ansprüche, **dadurch gekennzeichnet , dass** die Seitenwangen (4) beidseits des Kopfauflagebereichs (3b) lösbar am Auflageteil (1) bzw. an dessen Quersteg (3) festlegbar sind.

6. Vorrichtung nach Anspruch 5, **dadurch gekennzeichnet, dass** die Seitenwangen (4) mittels Klettverschluss (5) beidseits des Kopfauflagebereichs (3b) festlegbar sind.

7. Vorrichtung nach Anspruch 5 oder 6, **dadurch gekennzeichnet, dass** die Seitenwangen (4) quaderförmig ausgebildet sind.

8. Vorrichtung nach einem der vorhergehenden Ansprüche, **dadurch gekennzeichnet, dass** das Band (6) zur vertikalen Fixierung des Kopfes, insbesondere auf der Höhe der Stirn, seitlich am Quersteg (3) des Auflageteils (1) festlegbar ist.

9. Vorrichtung nach Anspruch 8, **dadurch gekennzeichnet, dass** das Band (6) mittels Klettverschluss (7) am Auflageteil (1) festlegbar ist.

10. Vorrichtung nach einem der vorhergehenden Ansprüche, **dadurch gekennzeichnet, dass** sie aus einem Schaumstoff besteht, der mit einem Überzug, insbesondere Kunstleder, versehen ist.
